# EUROPEAN PATENT APPLICATION

(11) **EP 2 502 917 A1**
(43) Date of publication of application: **26.09.2012**
(21) Application number: 11002220.9
(22) Date of filing: 17.03.2011
(51) Int. Cl.: C07D 233/58, A61K 31/4164, A61P 25/00

(54) **Solid state crystalline forms of 4-(2-ethyl-5-fluoro-2,3-dihydro-1H-inden-2-yl)-1H-imidazole**

(71) Applicant: Santhera Pharmaceuticals (Schweiz) AG, 4410 Liestal (CH)
(72) Inventor: Hausmann, Rudolf, 4052 Basel (CH); Hanbauer, Martin, 4022 Linz (AT); Stelmakh, Andriy, 4022 Linz (AT); Voglhofer, Bernhard, 4222 Sankt Georgen an der Gusen (AT); Rager, Timo, 5027 Herznach (CH); Blatter, Fritz, 4153 Reinach (CH)
(74) Representative: Grünecker, Kinkeldey, Stockmair & Schwanhäusser

(57) **Abstract**

The present invention describes novel crystalline forms of 4-(2-ethyl-5-fluoroindan-2-yl)-1H-imidazole, i.e. fipamezole and their use in the treatment of neurological diseases.

## Description

The present invention relates to crystalline forms of 4-(2-Ethyl-5-fluoro-2,3-dihydro-1H-inden-2-yl)-1*H*-imidazole hydrochloride, i.e. fipamezole hydrochloride. It further relates to the use of the crystalline forms of 4-(2-Ethyl-5-fluoro-2,3-dihydro-1H-inden-2-yl)-1*H-*imidazole hydrochloride in the treatment of neurological diseases.

Polymorphism is defined as the ability of a substance to exist as two or more crystalline phases that have different arrangement and/or conformations of the molecule in the crystal lattice. Different polymorphs may differ in their physical properties such as melting point, solubility, X-ray diffraction patterns etc. Although those differences disappear once the compound is dissolved, they can appreciably influence pharmaceutically relevant properties of the solid forms, such as handling properties, dissolution rate and stability. These properties can significantly influence the processing, shelf life and pharmaceutical composition of pharmaceutical formulations. Polymorphic forms of a compound can be distinguished in the laboratory by a number of analytical methods such as X-ray powder diffraction (XRPD), differential scanning calorimetry (DSC), infrared spectroscopy (IR) and FT-Raman spectroscopy (FT-RS).

Different crystalline forms may further exhibit different bioavailability patterns.

Fipamezole is a highly selective and long-acting antagonist of α₂.adrenoceptors. The compound is such valuable in the treatment of neurological disorders. Its preparation has been earlier described in WO-A-9313074. However, the preparation method described therein leads to a product (fipamezole hydrochloride) which leaves a desire for improvement as regards the stability thereof.

Accordingly, there remains a need in the art for well defined and stable forms of fipamezole hydrochloride which allow easy and reproducible production and handling of pharmaceutical formulations in combination with an extended shelf life of the medicament.

Surprisingly, it has been found that novel polymorphic forms of fipamezole hydrochloride meet the above mentioned need.

Thus, the present invention relates to crystalline forms A, B, B*, C, D and E of fipamezole hydrochloride. It further relates to processes for preparing said crystalline forms and to the use thereof in the treatment of neurological disorders.

### Brief description of the drawings

Figure 1 is a characteristic X-ray powder diffraction pattern of form A.
Figure 2 is a characteristic X-ray powder diffraction pattern of form B.
Figure 3 is a characteristic X-ray powder diffraction pattern of form B*.
Figure 4 is a characteristic X-ray powder diffraction pattern of form C.
Figure 5 is a characteristic X-ray powder diffraction pattern of form D.
Figure 6 is a characteristic X-ray powder diffraction pattern of form E.

Fipamezole hydrochloride has been described for the first time in WO-A-9313074 and has the following structure.

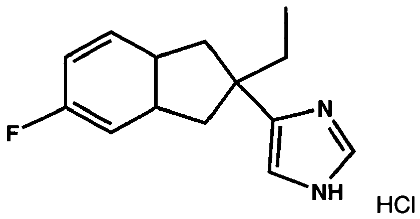

WO-A-9313074 further discloses a method for the preparation of fipamezole. The product obtained in said reference has been isolated as the hydrochloride salt prepared in ethyl acetate and characterised as having a melting point of 152 - 154 °C. The reference is, however, silent about the physical appearance of the described product.

The present invention relates to polymorphic forms of 4-(2-Ethyl-5-fluoro-2,3-dihydro-1H-inden-2-yl)-1*H*-imidazole (INN: fipamezole) hydrochloride which are designated as forms A, B, B*, C, D and E.

In one aspect, the present invention relates to a crystalline polymorph form of 4-(2-Ethyl-5-fluoro-2,3-dihydro-1H-inden-2-yl)-1*H*-imidazole hydrochloride or a hydrate thereof characterized in that it exhibits an X-ray powder diffraction pattern comprising characteristic peaks in degrees 2 Θ ± 0.2 at 12.0, 16.7, 17.5, 18.8, 19.1, 22.1, 22.4, 24.2, 24.8, 26.2, 26.9 and 27.7.
Said form has been designated as form A.

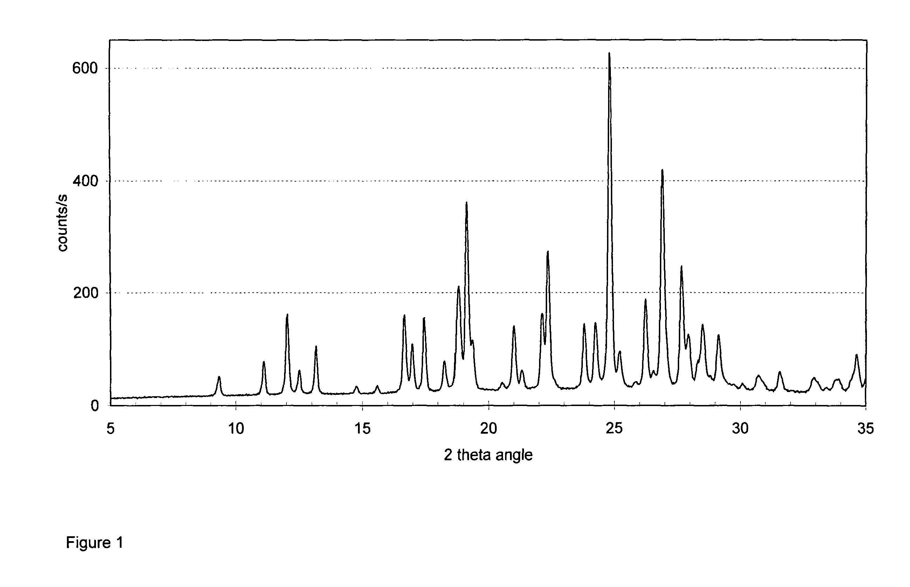

In a preferred embodiment, form A exhibits a XRPD pattern as substantially shown in Figure 1. The diffractogram was obtained on a Bruker D8 Advance using Cu K_{α} radiation.

In a further preferred embodiment, Form A has a melting point of 176 °C as confirmed by differential scanning calorimetry (DSC) on a Perkin Elmer DSC 7 with a heating rate of 10°C/min.

In a further aspect, the present invention relates to a crystalline polymorph form of 4-(2-Ethyl-5-fluoro-2,3-dihydro-1H-inden-2-yl)-1*H*-imidazole hydrochloride or a hydrate thereof characterized in that it exhibits an X-ray powder diffraction pattern comprising characteristic peaks in degrees 2 Θ ± 0.2 at 9.4, 18.7, 19.3, 22.4, 24.9, 26.4, 27.0, 27.8, 28.2, 28.8 and 36.0.
Said form has been designated as form B.

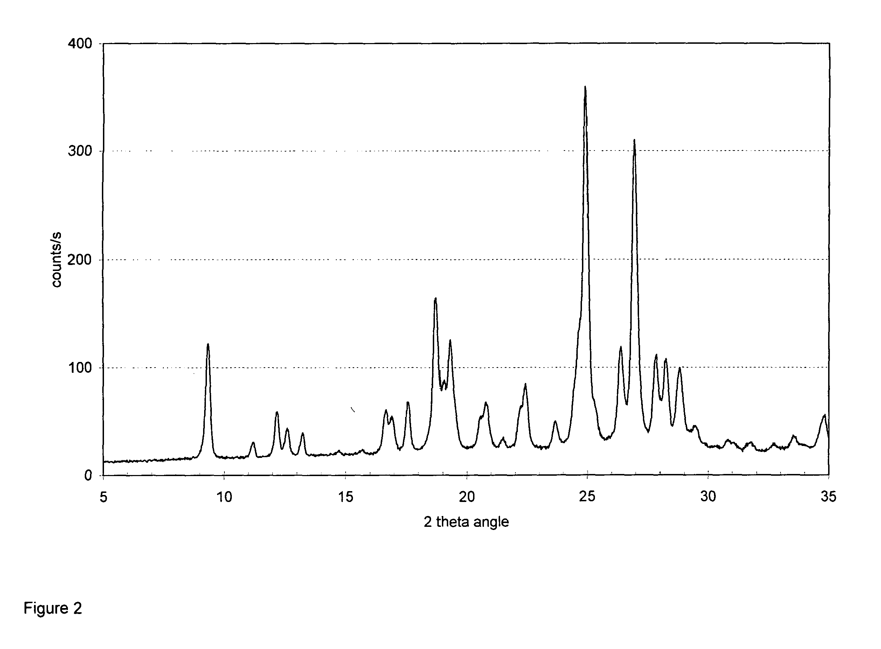

In a preferred embodiment, form B exhibits a XRPD pattern as substantially shown in Figure 2. The diffractogram was obtained on a Bruker D8 Advance using Cu K_{α} radiation.

In a further preferred embodiment, form B has a melting point of 170 °C as confirmed by differential scanning calorimetry (DSC) on a Perkin Elmer DSC 7 with a heating rate of 10°C/min.

In yet a further aspect, the present invention relates to a crystalline polymorph form of 4-(2-Ethyl-5-fluoro-2,3-dihydro-1H-inden-2-yl)-1*H*-imidazole hydrochloride or a hydrate thereof characterized in that it exhibits an X-ray powder diffraction pattern comprising characteristic peaks in degrees 2 Θ ± 0.2° at 12.2, 13.3, 16.8, 17.0, 17.6, 19.0, 19.3, 19.6, 22.4, 23.8, 24.4, 25.0, 26.4, 27.0, 27.9 and 28.7.
Said form has been designated as form B*.

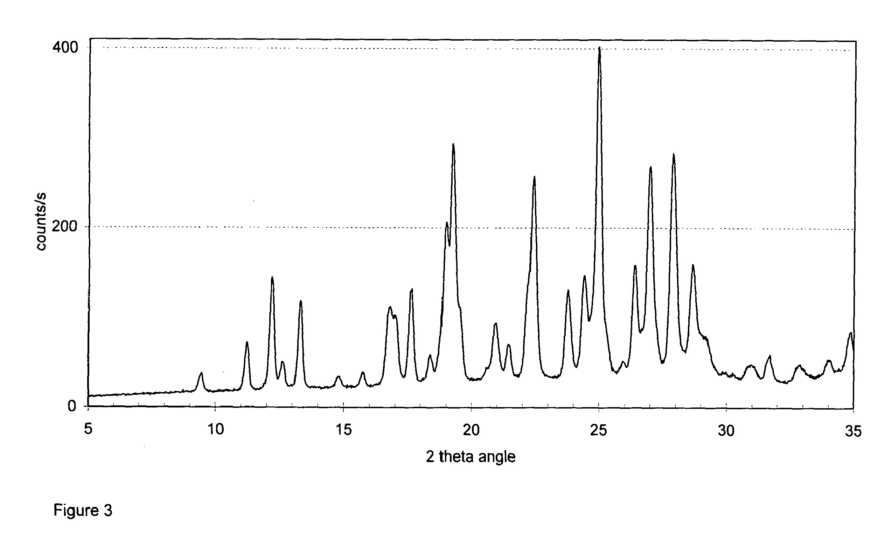

In a preferred embodiment, form B* exhibits a XRPD pattern as substantially shown in Figure 3. The diffractogram was obtained on a Bruker D8 Advance using Cu K_{α} radiation.

In a further aspect, the present invention relates to a crystalline polymorph form of 4-(2-Ethyl-5-fluoro-2,3-dihydro-1H-inden-2-yl)-1*H*-imidazole hydrochloride or a hydrate thereof characterized in that it exhibits an X-ray powder diffraction pattern comprising characteristic peaks in degrees 2 Θ ± 0.2° at 9.3, 18.7, 24.6, 24.9, 26.3, 26.9 and 28.2.
Said form has been designated as form C

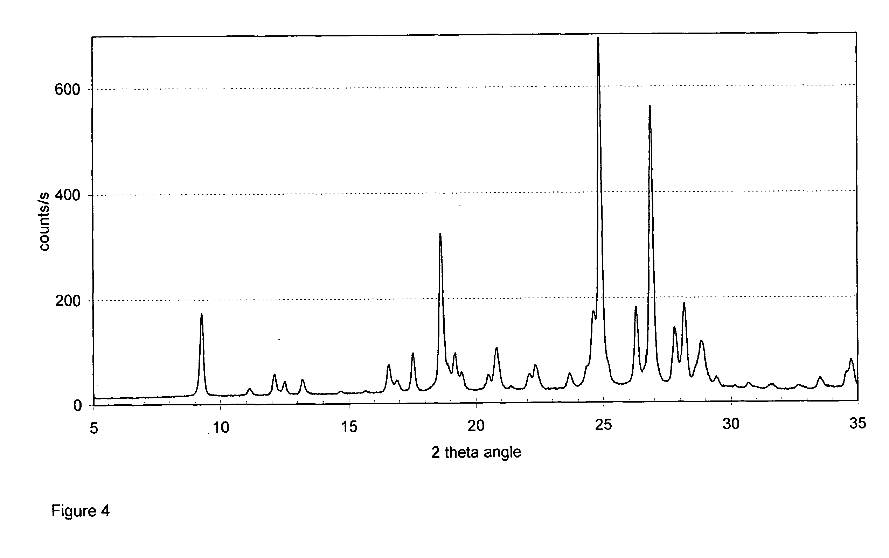

In a preferred embodiment, form C exhibits a XRPD pattern as substantially shown in Figure 4. The diffractogram was obtained on a Bruker D8 Advance using Cu K_{α} radiation.

In a further preferred embodiment, form C has a melting point of 173 °C as confirmed by differential scanning calorimetry (DSC) on a Perkin Elmer DSC 7 with a heating rate of 10°C/min.

In a further aspect, the present invention relates to a crystalline polymorph form of anhydrous 4-(2-Ethyl-5-fluoro-2,3-dihydro-1H-inden-2-yl)-1*H*-imidazole hydrochloride characterized in that it exhibits an X-ray powder diffraction pattern comprising characteristic peaks in degrees 2 Θ ± 0.2 Θ at 10.1, 13.2, 14.0, 15.0, 15.4, 16.5, 18.1, 19.9, 20.0, 20.3, 20.8, 22.5, 22.7, 23.1, 23.2, 24.1 and 27.4.
Said form has been designated as form D.

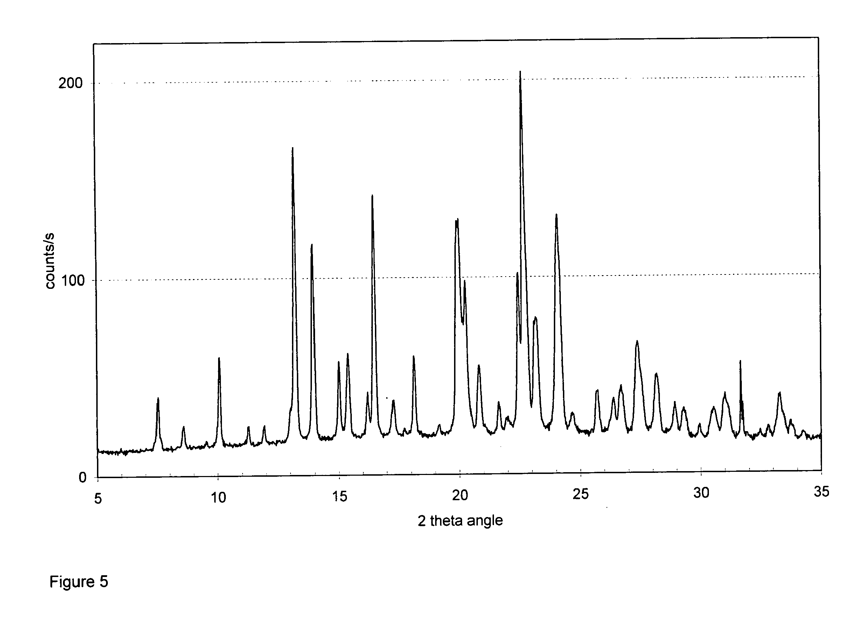

In a preferred embodiment, form D exhibits a XRPD pattern as substantially shown in Figure 5. The diffractogram was obtained on a Bruker D8 Advance using Cu K_{α} radiation.

In a further preferred embodiment, form D has a melting point of 189 - 190 °C as confirmed by differential scanning calorimetry (DSC) on a Perkin Elmer DSC 7 with a heating rate of 10°C/min.

In yet a further aspect, the present invention relates to a crystalline polymorph form of 4-(2-ethyl-5-fluoroindan-2-yl)-1*H*-imidazole hydrochloride or a solvate thereof characterized in that it exhibits an X-ray powder diffraction pattern comprising characteristic peaks in degrees 2 Θ ± 0.2 at 17.4, 18.7, 19.4, 19.8, 24.8, 26.3 and 27.0.
Said form has been designated as form E.

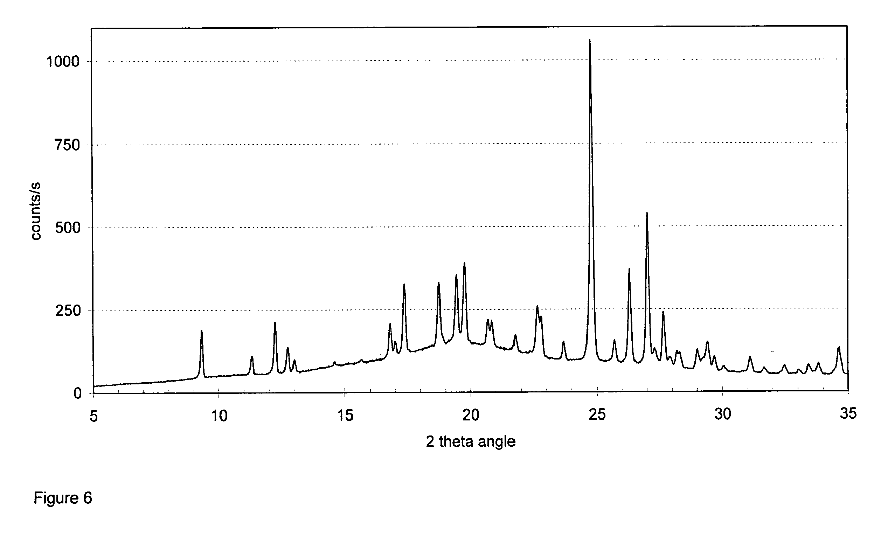

In a preferred embodiment, form E exhibits a XRPD pattern as substantially shown in Figure 6. The diffractogram was obtained on a Bruker D8 Advance using Cu K_{α} radiation.

It has been found that forms B, B*, C, D and E can transform into form A. Thus, forms B, B*, B, D and E can be used as intermediates in the preparation of form A. Accordingly, in the present invention also relates to the use of forms B, B*, C, D and E as intermediates in the preparation of form A. In a preferred embodiment, form C is used as the intermediate for preparing form A.

Forms A and D exhibit a high thermodynamic stability with form D having the higher melting temperature but lower melting enthalpy. Both forms can be converted into each other, accordingly, forms A and D represent an enantiotropic pair of polymorphs whereby form A is the low temperature form and form D the high temperature form.

Thus, forms A and D are particularly suited for the preparation of pharmaceutical formulation with an extended shelf life in comparison to pharmaceutical formulations presently under clinical investigation.

In a more preferred embodiment the present invention therefore relates to crystalline polymorph forms A and D of 4-(2-Ethyl-5-fluoro-2,3-dihydro-1H-inden-2-yl)-1*H*-imidazole hydrochloride, most preferably the present invention relates to form A.

Polymorphic form A can be prepared from forms B, B* and C. The preparation method comprises the steps of suspending one of forms B, B* and C in an aqueous medium and stirring the resulting suspension for a period of one hour up to three weeks, preferably up to two weeks.

In a preferred embodiment in combination with any embodiment above or below, the stirring is performed at a temperature from 0°C to 100°C, more preferably from 10°C to 80°C.

In a further preferred embodiment in combination with any embodiment above or below, the aqueous medium comprises an organic solvent in an amount of 10 % wt to 90 % wt, preferably in an amount of 25 % wt to 90 %.wt. The organic solvent may be any organic solvent which is miscible with water. Preferably, the organic solvent is selected from the group consisting of acetone, methanol, ethanol, 2-propanol, acetonitrile, methyl ethyl ketone, 1-propanol, 1-butanol, tetrahydrofuran or mixtures thereof.

Alternatively, form A can be prepared by stirring a suspension of form D in water at ambient temperature for a period of 1 hour up to two weeks. As a further alternative, form A can be prepared using form D as starting material by stirring a suspension of form D in water at ambient temperature for a period of 1 hour up to two weeks.

The solid form A obtained by using any of forms A, B, B*, C, D and E as starting material in the methods described above can be isolated by suitable methods known in the art such as filtration or centrifugation. Further work-up of the isolated solid such as drying or milling is optionally employed. Preferably, form A is dried to a water content of up to about 3 %wt.

Polymorphic forms B, B* and C can be obtained in a solution crystallisation process using the fipamezole hydrochloride salt of WO-A-9313074 as the starting material. The fipamezole hydrochloride is suspended in an organic solvent, water or mixtures thereof followed by heating the suspension to 50 - 150°C, preferably 70 - 130°C. The reaction mixture is subsequently cooled and the obtained solid is filtered off. Suitable organic solvents are 2-propanol, 1-butanol, acetone, acetonitrile, methanol, ethanol and ethyl acetate. The choice of the solvent(s) determines the resulting polymorphic form. The product is usually dried to a water content of 0 to about 4 % wt.

Polymorphic form D is preferably prepared by heating form C under protective gas atmosphere to a temperature of 165°C to 195°C for a period of 5 min. to 2 hours. As a result, form D is obtained as anhydrous crystalline product.

Polymorphic form E is prepared by suspending form C in an organic solvent such as 2-propanol and stirring the resulting suspension for a period of 1 to 10 days at ambient temperature.

The polymorphic forms A, B, B*, C, D and E, more preferably the polymorphic forms A, B and D and most preferably the polymorphic form A are suitable for use in the treatment of neurological diseases such as Alzheimer Disease and other diseases leading to cognitive deficits, Parkinson Disease, dyskinasia in Parkinson Disease, neurogenic orthostatic hypotension, Huntington Disease, schizophrenia or depression.

The effective dosage of the active ingredient employed may vary depending on the particular compounds employed, the mode of administration, the condition being treated and the severity of the condition being treated. Such dosage may be ascertained readily by a person skilled in the art. In humans, fipamezole hydrochloride is preferably administered in a dosage range of 10 mg/day to 600 mg/day, more preferably in a dosage range of 100 mg/day to 500 mg/day and most preferred in a dosage range of 250 mg/day to 400 mg/day.

Further, fipamezole hydrochloride is preferably administered at least once a day, preferably for at least 3 months, more preferably for at least 6 months, most preferably for 6 months to 12 months to observe the initial amelioration of the disease symptoms. For maintenance of the therapeutic effect prolonged treatment is recommended; the preferred treatment is lifelong.

Any suitable route of administration may be employed for providing a mammal, especially a human, with an effective dosage of a polymorphic form of fipamezole hydrochloride. The modes of administration include rectal, topical, ocular, pulmonary, oral, oromucosal, intraperitoneal (i.p.), intravenous (i.v.), intramuscular (i.m.), intracavernous (i.c.), parenteral, intranasal and transdermal. Preferred modes of administration are oral, oromucosal, intraperitoneal, intravenous, intramuscular, intracavernous, parenteral, intranasal, and transdermal, whereas the oromucosal administration is the even more preferred mode of administration. The oromucosal route is an administration route wherein the formulation containing the active ingredient is designed in a way that the active ingredient is absorbed primarily at one or more sites on the oral mucosa e.g. a sublingual formulation. In contrast, oral formulations may be designed to facilitate swallowing of the active ingredient. Uptake of the active ingredient will then take place in the gastrointestinal tract.

The active ingredient is preferably formulated into a dosage form prior to administration. The dosage forms include, e.g., tablets, pills, granules, powders, lozenges, sachets, cachets, elixirs, aqueous and oil-based suspensions, emulsions, dispersions, solutions such as sterile injectable solutions, syrups, aerosols (as a solid or in a liquid medium), capsules such as soft and hard gelatin capsules, suppositories, sterile packaged powders, troches, creams, ointments and aerosols. Tablets which allow the application of the oromucosal route, such as orally disintegrating tablets (ODT) are most preferred.

For oral application suitable preparations are in the form of tablets, sugar-coated pills, capsules, granular powders, drops, juices and syrups, while for parenteral, topical and inhalative application suitable forms are solutions, suspensions, easily reconstitutable dry preparations as well as sprays.

Accordingly, the polymorphic forms of fipamezole hydrochloride may be combined with any suitable pharmaceutical carrier. The pharmaceutical preparations for use in accordance with the present invention may be prepared by normal procedures using well-known and readily available ingredients. Such ingredients can be excipients, fillers, solvents, diluents, dyes and/or binders.

In making the formulations, a polymorphic form of fipamezole hydrochloride is usually mixed with a carrier, or diluted by a carrier, or enclosed with a carrier, which may be in the form of a capsule, cachet, paper or other container.

When the carrier serves as a diluent, it may be a solid, semi-solid, or liquid material, which acts as a vehicle, excipient or medium for the active ingredient.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate, gelatine, aspartame, glycine, sucrose octaacetate and mineral oil.

The formulations can additionally include lubricating agents, wetting agents, emulsifying and suspending agents, preserving agents, sweetening agents and/or flavoring agents.

The choice of auxiliary substances as well as the amounts thereof to be used depends on whether the medicinal drug is to be administered orally, intravenously, intraperitoneally, intradermally, intramuscularly, intranasally, buccally, sublingually or topically.

The polymorphic forms of fipamezole hydrochloride may be formulated so as to provide quick, sustained or delayed release of the active ingredient after administration to the patient. To this end, fipamezole hydrochloride can for example be administered in a sustained-release substance, in dissolved form or in a plaster, optionally with the addition of agents promoting penetration of the skin, and are suitable as percutaneous application preparations. Forms of preparations that can be used orally or percutaneously may produce a delayed release of the compounds.

Fipamezole hydrochloride is toxically safe which means that it can be used as a pharmaceutical active agent in a medicament.

The following examples further illustrate the invention.

### Experimental Procedures

***X-ray Powder Diffraction (XRPD).*** The measurements were carried out with a Bruker D8 Advance powder X-ray diffractometer using Cu Kα radiation in the Bragg-Brentano reflection geometry. Generally, the 2θ values are accurate within an error of ± 0.1-0.2°. The relative peak intensities can vary considerably for different samples of the same crystalline form because of different preferred orientations of the crystals. The samples were prepared without any special treatment other than the application of slight pressure to get a flat surface. The form E sample was measured under a Kapton film in order to prevent it from drying after crystallization. Silicon single crystal sample holders of either 0.5 mm or 0.1 mm depth and 12 mm cavity diameter were used. The tube voltage and current were 40 kV and 40 mA, respectively. The X-ray diffractometer is equipped with a LynxEye detector. A variable divergence slight was used with a 3° window. The step size was 0.02 °2θ with a step time of 37 seconds. The samples were rotated at 0.5 rps during the measurement. Data peak tables were created by the Philips X'Pert HighScore Plus software using background correction partial profile fitting, the copper K_{α2} lines were removed.

***Differential Scanning Calorimetry (DSC):*** DSC measurements were carried out on a Perkin Elmer DSC-7. The samples were placed into gold crucibles that were sealed under nitrogen. The measurements were performed with a heating rate of 10 or 20 °C min⁻¹ over the temperature range from -50 °C to about 200°C.

### Examples

### Example 1: Preparation of form A with form C as starting material

116 mg of fipamezole hydrochloride form C are suspended in 1 mL H₂O and the resulting suspension is stirred at room temperature for 2 weeks. Then the crystalline solid is separated by filtration and without further drying investigated by powder X-ray diffraction. A new, and for form A characteristic powder X-ray diffraction pattern as displayed in Figure 1 is obtained which exhibits X-ray diffraction reflexions as listed in Table 1.

**Table 1:**

| Pos. [°20.] | d-spacing [Å] | Qualitative Intensity |
|---|---|---|
| 9.3 | 9.5 | vw |
| 11.1 | 8.0 | w |
| 12.0 | 7.3 | m |
| 12.5 | 7.1 | vw |
| 13.2 | 6.7 | w |
| 14.8 | 5.99 | vw |
| 15.6 | 5.68 | vw |
| 16.7 | 5.31 | m |
| 17.0 | 5.22 | w |
| 17.5 | 5.08 | m |
| 18.2 | 4.86 | vw |
| 18.8 | 4.71 | m |
| 19.1 | 4.63 | s |
| 19.4 | 4.58 | w |
| 21.0 | 4.23 | w |
| 22.1 | 4.02 | m |
| 22.4 | 3.97 | s |
| 23.8 | 3.74 | w |
| 24.2 | 3.67 | m |
| 24.8 | 3.59 | vs |
| 25.2 | 3.53 | w |
| 26.2 | 3.40 | m |
| 26.9 | 3.31 | s |
| 27.7 | 3.22 | m |
| 28.5 | 3.13 | w |
| 29.1 | 3.06 | w |
| 34.6 | 2.59 | w |

| | | |
|---|---|---|
| vs: very strong, s: strong, m: medium, w: weak, vw: very weak intensity | | |

### Example 2: Preparation of form A with form C as starting material

6.01 g of fipamezole hydrochloride form C are suspended in a mixture of 1.79 g H₂O, and 12.24 g acetone. The suspension is heated to 60 °C at a rate of 0.1 K/min, kept at this temperature for 30 min, cooled back to 0 °C at a cooling rate of 0.1 K/min and stirred at this temperature for about 1 h. The solid is then filtered off on a glass filter and shortly dried by pulling air through the filter using vacuum until a free-flowing powder was obtained. Investigation of the obtained material by powder X-ray diffraction shows the characteristic powder X-ray diffraction pattern for form A as displayed in Figure 1.

### Example 3: Preparation of form A with form B as starting material

A suspension of 118 mg fipamezole hydrochloride form B in 1 mL H₂O is stirred at room temperature for two days. The solid is filtered off by centrifuge filtration and analyzed by powder X-ray diffraction, which reveals the characteristic powder X-ray diffraction pattern for form A as displayed in Figure 1. An aliquot of the solution is diluted with water by a factor of 500 and analyzed by UVNis spectroscopy. The aqueous solubility was determined by evaluation of the extinction at 270 nm and found to be about 46 g/L.

### Example 4: Preparation of form A with form B* as starting material

A suspension of 97 mg fipamezole hydrochloride form B* in 0.5 mL H₂O is stirred at room temperature for two days. The solid is filtered off by centrifuge filtration and analyzed by powder X-ray diffraction, which reveals the characteristic powder X-ray diffraction pattern for form A as displayed in Figure 1. An aliquot of the solution is diluted with water by a factor of 500 and analyzed by UVNis spectroscopy. The aqueous solubility was determined by evaluation of the extinction at 270 nm and found to be about 43 g/L.

### Example 5: Preparation of form A with form D as starting material

A suspension of 110 mg fipamezole hydrochloride form D in 0.5 mL H₂O is stirred at room temperature for two days. The solid is filtered off by centrifuge filtration and analyzed by powder X-ray diffraction, which reveals the characteristic powder X-ray diffraction pattern for form A as displayed in Figure 1. An aliquot of the solution is diluted with water by a factor of 500 and analyzed by UVNis spectroscopy. The aqueous solubility was determined by evaluation of the extinction at 270 nm and found to be about 46 g/L.

### Example 6: Preparation of form A with form E as starting material

A suspension of 150 mg fipamezole hydrochloride form E in 1 mL H₂O is stirred at room temperature for two days. The solid is filtered off by centrifuge filtration and analyzed by powder X-ray diffraction, which reveals the characteristic powder X-ray diffraction pattern for form A as displayed in Figure 1.

### Example 7: Preparation of form B in a solution crystallization process

200 mg of fipamezole hydrochloride is dissolved in 1 mL 2-propanol at reflux temperature. The clear solution is removed from the heatbath and 10 mL acetone is added. The mixture is slightly stirred at room temperature until crystallization is observed and the filtration is performed after stirring overnight at room temperature. The solid product is recovered by filtration and short drying in air at room temperature. Investigation of the obtained material by powder X-ray diffraction shows the characteristic powder X-ray diffraction pattern for form B as displayed in Figure 2.

The X-ray diffraction reflexions of form B are listed in Table 2 below.

**Table 2**

| Pos. [°2θ.] | d-spacing [Å] | Qualitative Intensity |
|---|---|---|
| 9.4 | 9.5 | m |
| 11.2 | 7.9 | vw |
| 12.2 | 7.3 | w |
| 12.6 | 7.0 | w |
| 13.2 | 6.7 | vw |
| 16.7 | 5.32 | w |
| 16.9 | 5.23 | w |
| 17.6 | 5.04 | w |
| 18.7 | 4.73 | s |
| 19.3 | 4.60 | m |
| 20.8 | 4.26 | w |
| 22.4 | 3.97 | m |
| 24.9 | 3.57 | vs |
| 26.4 | 3.38 | m |
| 27.0 | 3.31 | vs |
| 27.8 | 3.20 | m |
| 28.2 | 3.16 | m |
| 28.8 | 3.10 | m |
| 34.8 | 2.58 | w |
| 36.0 | 2.49 | m |

| | | |
|---|---|---|
| vs: very strong, s: strong, m: medium, w: weak, vw: very weak intensity | | |

### Example 8: Preparation of form B* in a solution crystallization process

4.01 g fipamezole hydrochloride is suspended in a mixture of 0.42 g H₂O and 15.61 g 2-propanol. The suspension is heated to 85 °C at 0.1 K/min, kept at this temperature for 30 min. A clear solution is obtained which is subsequently cooled to 0 °C at a cooling rate of 0.1 K/min. Stirring at 0°C is carried out for about one hour, before the solid is filtered off on a glass filter. Air is pulled through the filter by applying vacuum until a free-flowing powder was obtained. Investigation of the obtained material by powder X-ray diffraction shows the characteristic powder X-ray diffraction pattern for form B* as displayed in Figure 3.

The X-ray diffraction reflexions of form B* are listed in Table 3 below.

**Table 3:**

| Pos. [°2θ.] | d-spacing [Å] | Qualitative Intensity |
|---|---|---|
| 9.4 | 9.4 | vw |
| 11.2 | 7.9 | w |
| 12.2 | 7.2 | m |
| 12.6 | 7.0 | vw |
| 13.3 | 6.6 | m |
| 14.8 | 5.98 | vw |
| 15.7 | 5.63 | vw |
| 16.8 | 5.28 | m |
| 17.0 | 5.20 | m |
| 17.6 | 5.03 | m |
| 18.4 | 4.82 | vw |
| 19.0 | 4.67 | s |
| 19.3 | 4.60 | vs |
| 19.6 | 4.54 | m |
| 20.9 | 4.24 | w |
| 21.5 | 4.14 | w |
| 22.2 | 4.00 | shoulder |
| 22.4 | 3.96 | s |
| 23.8 | 3.74 | m |
| 24.4 | 3.64 | m |
| 25.0 | 3.56 | vs |
| 26.4 | 3.37 | m |
| 27.0 | 3.30 | s |
| 27.9 | 3.20 | vs |
| 28.7 | 3.11 | m |
| 34.9 | 2.57 | w |

| | | |
|---|---|---|
| vs: very strong, s: strong, m: medium, w: weak, vw: very weak intensity | | |

### Example 9: Preparation of form C in a solution crystallization process

152 mg of fipamezole hydrochloride is dissolved in 1 mL 1-butanol at ca. 100 °C. The vial with the clear solution is kept on the laboratory shelf and let cool to room temperature, then it is further cooled and stored at 5 °C. A white precipitate is formed after several hours, which is recovered by filtration and short drying in air at room temperature. Investigation of the obtained material by powder X-ray diffraction shows the characteristic powder X-ray diffraction pattern for form C as displayed in Figure 4.

The X-ray diffraction reflexions of form C are listed in Table 4 below.

**Table 4:**

| Pos. [°2θ.] | d-spacing [Å] | Qualitative Intensity |
|---|---|---|
| 9.3 | 9.53 | m |
| 11.1 | 7.95 | vw |
| 12.1 | 7.31 | vw |
| 12.5 | 7.08 | vw |
| 13.2 | 6.70 | vw |
| 16.6 | 5.34 | vw |
| 16.9 | 5.24 | vw |
| 17.5 | 5.06 | w |
| 18.7 | 4.75 | s |
| 19.2 | 4.63 | vw |
| 20.8 | 4.27 | w |
| 22.3 | 3.98 | vw |
| 23.7 | 3.76 | vw |
| 24.6 | 3.61 | m |
| 24.9 | 3.57 | vs |
| 26.3 | 3.39 | m |
| 26.9 | 3.31 | vs |
| 27.8 | 3.21 | w |
| 28.2 | 3.17 | m |
| 28.8 | 3.10 | w |
| 34.7 | 2.58 | vw |
| 36.0 | 2.50 | w |

| | | |
|---|---|---|
| vs: very strong, s: strong, m: medium, w: weak, vw: very weak intensity | | |

### Example 10: Preparation of form D

502 mg of fipamezole hydrochloride form C is heated under N₂ to 170-175 °C for one hour and 15 minutes. The agglomerated material is crushed in an agate mortar. Investigation of the obtained material by powder X-ray diffraction shows the characteristic powder X-ray diffraction pattern for form D as displayed in Figure 5 and the respective data are listed in Table 5.

**Table 5:**

| Pos. [°2θ.] | d-spacing [Å] | Qualitative Intensity |
|---|---|---|
| 7.5 | 11.7 | w |
| 8.6 | 10.3 | vw |
| 10.1 | 8.8 | m |
| 11.3 | 7.9 | vw |
| 11.9 | 7.4 | vw |
| 13.2 | 6.7 | vs |
| 14.0 | 6.3 | s |
| 15.0 | 5.89 | m |
| 15.4 | 5.75 | m |
| 16.2 | 5.47 | w |
| 16.5 | 5.37 | vs |
| 17.3 | 5.13 | w |
| 18.1 | 4.89 | m |
| 19.9 | 4.45 | s |
| 20.0 | 4.43 | s |
| 20.3 | 4.37 | s |
| 20.8 | 4.26 | m |
| 22.5 | 3.96 | s |
| 22.7 | 3.92 | vs |
| 23.1 | 3.84 | m |
| 23.2 | 3.83 | m |
| 24.1 | 3.69 | s |
| 27.4 | 3.25 | m |

| | | |
|---|---|---|
| vs: very strong, s: strong, m: medium, w: weak, vw: very weak intensity | | |

### Example 11: Preparation of form D

100 mg fipamezole hydrochloride form C is heated in an oil bath under N₂ to 190 °C for about 15 min. Some sintering but no melting is observed. The sample is cooled to room temperature under nitrogen and investigation of the obtained material by powder X-ray diffraction shows the characteristic powder X-ray diffraction pattern for form D as displayed in Figure 5. Differential scanning calorimetry of the obtained sample of form D shows a melting peak at 190°C with an enthalpy of fusion of about 91 J/g.

### Example 12: Preparation of form E.

A suspension of 216 mg fipamezole hydrochloride form C in 1 mL 2-propanol is stirred at room temperature for 5 days. The solid is filtered off (centrifuge filtration, 1 min at 3000 rpm) and prepared wet under Kapton foil for powder X-ray diffraction. The investigation of the obtained material by powder X-ray diffraction shows the characteristic powder X-ray diffraction pattern for form E as displayed in Figure 6. The respective X-ray diffraction reflexions are listed in Table 6 below.

**Table 6:**

| Pos. [°2θ.] | d-spacing [Å] | Qualitative Intensity |
|---|---|---|
| 9.3 | 9.5 | w |
| 11.3 | 7.8 | vw |
| 12.2 | 7.2 | w |
| 12.7 | 6.9 | vw |
| 13.0 | 6.8 | vw |
| 16.8 | 5.27 | w |
| 17.4 | 5.10 | m |
| 18.7 | 4.73 | m |
| 19.4 | 4.56 | m |
| 19.8 | 4.49 | m |
| 20.7 | 4.29 | w |
| 20.9 | 4.26 | w |
| 21.8 | 4.08 | vw |
| 22.6 | 3.92 | w |
| 22.8 | 3.90 | w |
| 24.8 | 3.59 | vs |
| 25.7 | 3.47 | vw |
| 26.3 | 3.39 | m |
| 27.0 | 3.30 | s |
| 27.6 | 3.22 | w |
| 34.6 | 2.59 | vw |
| 36.1 | 2.48 | w |

| | | |
|---|---|---|
| vs: very strong, s: strong, m: medium, w: weak, vw: very weak intensity | | |

## Claims

1. A crystalline polymorph form A of 4-(2-Ethyl-5-fluoro-2,3-dihydro-1H-inden-2-yl)-1*H* imidazole hydrochloride or a hydrate thereof **characterized in that** it exhibits an X-ray powder diffraction pattern comprising characteristic peaks in degrees 2 Θ ± 0.2° at 12.0, 16.7, 17.5, 18.8, 19.1, 22.1, 22.4, 24.2, 24.8, 26.2, 26.9 and 27.7.

2. The crystalline polymorph of claim 1 which exhibits an X-ray powder diffraction pattern substantially according to Figure 1.

3. A crystalline polymorph form B of 4-(2-Ethyl-5-fluoro-2,3-dihydro-1H-inden-2-yl)- 1H-imidazole hydrochloride or a hydrate thereof **characterized in that** it exhibits an X-ray powder diffraction pattern comprising characteristic peaks in degrees 2 Θ ± 0.2° at 9.4, 18.7, 19.3, 22.4, 24.9, 26.4, 27.0, 27.8, 28.2, 28.8 and 36.0.

4. The crystalline polymorph of claim 3 which exhibits an X-ray powder diffraction pattern substantially according to Figure 2.

5. A crystalline polymorph form B* of 4-(2-Ethyl-5-fluoro-2,3-dihydro-1H-inden-2-yl)-1H-imidazole hydrochloride or a hydrate thereof **characterized in that** it exhibits an X-ray powder diffraction pattern comprising characteristic peaks in degrees 2 Θ ± 0.2° at 12.2, 13.3, 16.8, 17.0, 17.6, 19.0, 19.3, 19.6, 22.4, 23.8, 24.4, 25.0, 26.4, 27.0, 27.9 and 28.7.

6. The crystalline polymorph of claim 5 which exhibits an X-ray powder diffraction pattern substantially according to Figure 3.

7. A crystalline polymorph Form C of 4-(2-Ethyl-5-fluoro-2,3-dihydro-1H-inden-2-yl)-1H-imidazole hydrochloride or a hydrate thereof **characterized in that** it exhibits an X-ray powder diffraction pattern comprising characteristic peaks in degrees 2 Θ ± 0.2° at 9.3, 18.7, 24.6, 24.9, 26.3, 26.9 and 28.2.

8. The crystalline polymorph of claim 7 which exhibits an X-ray powder diffraction pattern substantially according to Figure 4.

9. A crystalline polymorph form D of anhydrous 4-(2-Ethyl-5-fluoro-2,3-dihydro-1H-inden-2-yl)-1*H*-imidazole hydrochloride **characterized in that** it exhibits an X-ray powder diffraction pattern comprising characteristic peaks in degrees 2 Θ ± 0.2° at 10.1, 13.2, 14.0, 15.0, 15.4, 16.5, 18.1, 19.9, 20.0, 20.3, 20.8, 22.5, 22.7, 23.1, 23.2, 24.1 and 27.4.

10. The crystalline polymorph of claim 9 which exhibits an X-ray powder diffraction pattern substantially according to Figure 5.

11. A crystalline polymorph form E of 4-(2-Ethyl-5-fluoro-2,3-dihydro-1H-inden-2-yl)- 1H-imidazole hydrochloride or a solvate thereof **characterized in that** it exhibits an X-ray powder diffraction pattern comprising characteristic peaks in degrees 2 Θ ± 0.2° at 17.4, 18.7, 19.4, 19.8, 24.8, 26.3 and 27.0.

12. The crystalline polymorph of claim 11 which exhibits an X-ray powder diffraction pattern substantially according to Figure 6.

13. The compound of any of claims 1 to 12 for use in the treatment of neurological disorders.

14. The compound of claim 13 for use in the treatment of Dyskinesia in Parkinson Disease.

15. A pharmaceutical composition comprising a compound according to any of claims 1 to 12 and a pharmaceutically acceptable carrier.
